(19) Europäisches Patentamt
European Patent Office
Office européen des brevets

(11) Veröffentlichungsnummer: **0 301 197 B1**

(12) **EUROPÄISCHE PATENTSCHRIFT**

(45) Veröffentlichungstag der Patentschrift: **18.09.91**

(51) Int. Cl.5: **A61K 7/11**

(21) Anmeldenummer: **88108592.2**

(22) Anmeldetag: **30.05.88**

(54) **Haarwachs.**

(30) Priorität: **29.07.87 DE 3725080**

(43) Veröffentlichungstag der Anmeldung:
**01.02.89 Patentblatt 89/05**

(45) Bekanntmachung des Hinweises auf die
Patenterteilung:
**18.09.91 Patentblatt 91/38**

(84) Benannte Vertragsstaaten:
**DE ES FR GB IT**

(56) Entgegenhaltungen:
**EP-A- 0 172 713**
**DE-A- 1 467 825**
**US-A- 2 309 722**
**US-A- 3 458 624**

**PATENT ABSTRACTS OF JAPAN, Band 3, Nr.
100 (C-56), 24. August 1979, Seite 29 C 56; &
JP-A-54 76 835 (KANEBO K.K.) 19-06-1979**

**Handbuch der Kosmetika und Riechstoffe
Band 3 (1973) Seite 325**

(73) Patentinhaber: **Wella Aktiengesellschaft
Berliner Allee 65
W-6100 Darmstadt(DE)**

(72) Erfinder: **Gross, Paul
Forstweg 54
W-6100 Darmstadt(DE)**
Erfinder: **Flemming, Ernst
Königsberger Strasse 17
W-6056 Heusenstamm(DE)**

**Beschreibung**

Die Erfindung betrifft ein mit Wasser leicht ausspülbares Haarwachs.

Halbfeste Mittel zur Frisurengestaltung sind unter der Bezeichnung Stangenpomade oder Brillantine bereits seit langem bekannt. Diese Frisierhilfsmittel werden bevorzugt zur Behandlung des männlichen Haares verwendet und sollen ein glattes Anliegen der Haare auf dem Kopf gewährleisten. Weiterhin sollen diese Mittel dem Haar Glanz verleihen.

Zur Gestaltung der weiblichen Frisur werden hingegen überwiegend sogenannte Haarwachse benutzt. Da von derartigen Haarwachsen auch eine "festigende" Wirkung erwartet wird, müssen sie eine etwas festere Konsistenz als Brillantinen oder Pomaden aufweisen und insbesondere "klebende" Eigenschaften (das bedeutet hohe Kohäsionskräfte) besitzen.

Haarwachse bestehen in der Regel aus einem Gemisch von festen, halbfesten und flüssigen gesättigten Kohlenwasserstoffen, wie zum Beispiel Petrolatum, festes Paraffin oder Paraffinöle. Sie besitzen wachsartige und klebende sowie glanzgebende Eigenschaften und ermöglichen deshalb eine befriedigende Frisurengestaltung sowie die Behandlung und Formung einzelner Haarsträhnen. Aufgrund ihrer Zusammensetzung lassen sich derartige Haarwachse jedoch nur sehr schwer wieder aus dem Haar entfernen. Mit Wasser sind derartige Haarwachse nicht aus dem Haar entfernbar und selbst durch mehrmaliges Haarewaschen mit einem Haarshampoo werden sie nur unzureichend entfernt, so daß das Haar bei häufiger Anwendung dieser Haarwachse unansehnlich und "speckig" wird.

In der Literatur werden zwar mit Wasser entfernbare Brillantinen und Stangenpomaden beschrieben, jedoch besitzen diese Mittel nicht die gewünschten frisurengestaltenden und festigenden Eigenschaften. So wird beispielsweise im "Handbuch der Kosmetika und Riechstoffe", Band III (1973) von Hugo Janistyn, Seite 325 eine mit Wasser auswaschbare Stangenpomade beschrieben, die aus 60 Gewichtsprozent Polyethylenglykol (MG = 4000) und 40 Gewichtsprozent Ethylhexandiol besteht. Mit einer derartig zusammengesetzten Standgenpomade behandelte Haarsträhnen besitzen jedoch, wie Vergleichsversuche (siehe Beispiel 9) zeigen, weder einen befriedigenden Glanz noch eine ausreichende Formstabilität oder ein befriedigendes Standvermögen.

Es bestand deshalb die Aufgabe, ein mit Wasser leicht ausspülbares Haarwachs zur Verfügung zu stellen, das eine gute Frisurengestaltung sowie die individuelle Behandlung einzelner Haarsträhnen in gleicher Weise ermöglicht wie die bisher bekannten Haarwachse.

Überraschenderweise wurde nun gefunden, daß ein Haarwachs, welches dadurch gekennzeichnet ist, daß es eine Kombination von

(a) 10 bis 41 Gewichtsprozent Polyethylenglykol mit einem Molekulargewicht von 3000 bis 5000,

(b) 8 bis 25 Gewichtsprozent hydriertes Rizinusöl, welches mit 40 bis 60 Mol Ethylenoxid oxethyliert ist,

(c) 36 bis 51 Gewichtsprozent Glycerin und/oder Ethylhexandiol und/oder Polyethylenglykol mit einem Molekulargewicht von 100 bis 300 und

(d) 0 bis 15 Gewichtsprozent Wasser

enthält, die gestellte Aufgabe in hervorragender Weise erfüllt.

Eine bevorzugte Ausführungsform der Erfindung ist ein Haarwachs, das eine Kombination von

(a) 32 bis 41 Gewichtsprozent Polyethylenglykol mit einem Molekulargewicht von 3000 bis 5000,

(b) 8 bis 25 Gewichtsprozent hydriertes Rizinusöl, welches mit 40 bis 45 Mol Ethylenoxid oxethyliert ist,

(c) 36 bis 51 Gewichtsprozent Glycerin und/oder Ethylhexandiol und/oder Polyethylenglykol mit einem Molekulargewicht von 100 bis 300 und

(d) 0 bis 10 Gewichtsprozent Wasser

enthält.

Eine weitere bevorzugte Ausführungsform der Erfindung ist ein Haarwachs, das eine Kombination von

(a) 10 bis 32 Gewichtsprozent Polyethylenglykol mit einem Molekulargewicht von 3000 bis 5000,

(b) 8 bis 25 Gewichtsprozent hydriertes Rizinusöl, welches mit 40 bis 60 Mol Ethylenoxid oxethyliert ist,

(c) 36 bis 51 Gewichtsprozent Glycerin und/oder Ethylhexandiol und/oder Polyethylenglykol mit einem Molekulargewicht von 100 bis 300,

(d) 0 bis 15 Gewichtsprozent Wasser und

(e) 5 bis 25 Gewichtsprozent eines Perlglanzpigmentes enthält.

Besonders bevorzugte Ausführungsformen der Erfindung sind Haarwachse, die entweder eine Kombination von

(a) 35 bis 39 Gewichtsprozent Polyethylenglykol mit einem Molekulargewicht von 4000,

(b) 11 bis 18 Gewichtsprozent hydriertes Rizinusöl, welches mit 40 bis 45 Mol Ethylenoxid oxethyliert ist,

(c) 39 bis 46 Gewichtsprozent Glycerin und/oder Ethylhexandiol und/oder Polyethylenglykol mit einem Molekulargewicht von 200 und

(d) 2 bis 8 Gewichtsprozent Wasser

oder eine Kombination von

(a) 10 bis 25 Gewichtsprozent Polyethylenglykol, mit einem Molekulargewicht von 4000,

(b) 11 bis 18 Gewichtsprozent hydriertes Rizinusöl, welches mit 40 bis 45 Ethylenoxid oxethyliert ist,

(c) 39 bis 46 Gewichtsprozent Glycerin und/oder Ethylhexandiol und/oder Polyethylenglykol mit einem Molekulargewicht von 200,

(d) 2 bis 12 Gewichtsprozent Wasser und

(e) 10 bis 20 Gewichtsprozent eines Perlglanzpigmentes

enthalten.

Das erfindungsgemäße Haarwachs ermöglicht aufgrund seiner wachsartigen Konsistenz und seiner "klebenden" (das heißt kohäsiven) Eigenschaften eine individuelle Frisurengestaltung sowie die Behandlung einzelner Haarsträhnen. Beispielsweise können einzelne Haarsträhnen mit diesem Haarwachs in gleicher Weise wie mit den bisher üblichen Haarwachsen behandelt werden, so daß sie dauerhaft und elastisch von der Kopfhaut abstehen. Das so behandelte Haar zeichnet sich durch einen ausgeprägten Glanz sowie eine hohe Formstabilität der erstellten Frisur aus. Der entscheidende Vorteil des erfindungsgemäßen Haarwachs gegenüber den bisher üblichen Haarwachsen auf Kohlenwasserstoffbasis ist jedoch, daß das neue Haarwachs mit Wasser leicht und völlig rückstandsfrei entfernt werden kann.

Zusätzlich zu den vorstehend aufgeführten Komponenten (a) bis (e) kann das Haarwachs weitere für ein derartiges Mittel übliche und bekannte Zusatzstoffe enthalten. Beispiele für derartige Zusatzstoffe sind Parfümöle, haarpflegende Wirkstoffe und Konservierungsmttel, wie beispielsweise Formaldehyd, Salicylsäure, Parahydroxybenzoesäureester, Benzoesäure, Mandelsäure, Polyhexamethylen-biguanid-hydrochlorid oder Isothiazolinonderivate. Die Konservierungsstoffe können dem Haarwachs in einer Menge von etwa 0,01 bis 1 Gewichtsprozent zugesetzt werden, während die Parfümöle in einer Menge von etwa 0,01 bis 2 Gewichtsprozent in dem Haarwachs enthalten sein können.

Weiterhin können dem Haarwachs übliche kosmetische Haarfarbstoffe, wie zum Beispiel C.I. Pigment Red 4 (C.I. 12 085), C.I. Pigment Green (C.I. 74 260) und C.I. Vat Blue 4 (C.I. 69 800), und/oder Perlglanzpigmente beispielsweise CTFA-Titandioxid/Mica/Eisenoxid, CTFA-Titandioxid/Mica/Carmin, CTFA-Titandioxid/mica/Eisenferrocyanid, CTFA-Titandioxid/Mica/Chromoxidgrün und CTFA-Titandioxid/Mica zugesetzt werden. Die Haarfarbstoffe werden hierbei in einer Menge von 0,1 bis 6 Gewichtsprozent, vorzugsweise von 0,5 bis 4 Gewichtsprozent, eingesetzt, während die Perlglanzpigmente in einer Menge von 1 bis 25 Gewichtsprozent, vorzugsweise von 10 bis 20 Gewichtsprozent, in dem erfindungsgemäßen Haarwachs enthalten sein können.

Mit einem derartigen Haarwachs ist eine gleichzeitige Anfärbung der behandelten Haare möglich, wodurch spezielle modische Effekte, beispielsweise die Anfärbung einzelner Haarsträhnen, erzielt werden können.

Die vorstehend aufgeführen Farbstoffe beziehungsweise Perlglanzpigmente sowie weitere geeignete Färbemittel werden im Colour-Index, 3rd Edition, Volume 3, The Society of Dyers and Colourists, Großbritannien (1971), beziehungsweise CTFA-Cosmetic Ingredient Dictionary, 3rd Edition, The Cosmetic, Toiletry and Fragrance Association Inc., USA (1982), beschrieben.

Die nachfolgenden Beispiele sollen den Gegenstand der Erfindung näher erläutern.

**Beispiele**

**Beispiel 1: Haarwachs**

```
37,5 g Polyethylenglykol (MG = 4000)
26,0 g Glycerin
20,0 g Polyethylenglykol (MG = 200)
10,8 g hydriertes Rizinusöl, mit 40 bis 45
       Mol Ethylenoxid oxethyliert (Cremo-
       phor® RH 40, BASF Aktiengesell-
       schaft)
 0,3 g Parfumöl
 5,4 g Wasser
100,0 g
```

**Beispiel 2: Haarwachs**

```
39,0 g Polyethylenglykol (MG = 4000)
38,7 g Glycerin
14,1 g hydriertes Rizinusöl, mit 40 bis 45
       Mol Ethylenoxid oxethyliert (Cremo-
       phor® RH 40, BASF Aktiengesell-
       schaft)
 0,3 g Parfümöl
 7,9 g Wasser
100,0 g
```

**Beispiel 3: Haarwachs**

```
45,0 g Ethyhexandiol
35,0 g Polyethylenglykol (MG = 4000)
18,0 g hydriertes Rizinusöl, mit 40 bis 45
       Mol Ethylenoxid oxethyliert (Cremo-
       phor® RH 40, BASF Aktiengesell-
       schaft)
```

```
 2,0 g Wasser
100,0 g
```

**Beispiel 4: Das Haar rot anfärbendes Haarwachs**

EP 0 301 197 B1

42,6 g Glycerin

35,0 g Polyethylenglykl (MG = 4000)

11,7 g hydriertes Rizinusöl, mit 40 bis 45
Mol Ethylenoxid oxethyliert (Cremophor® RH 40, BASF Aktiengesellschaft)

2,5 g C.I. Pigment Red 4 (C.I. 12 085)

8,2 g Wasser

100,0 g

**Beispiel 5: Das Haar grün anfärbendes Haarwachs**

35,0 g Polyethylenglykol (MG = 4000)

25,4 g Glycerin

20,0 g Ethylhexandiol

11,7 g hydriertes Rizinusöl, mit 40 bis 45
Mol Ethylenoxid oxethyliert (Cremophor® RH 40, BASF Aktiengesellschaft)

2,5 g C.I. Pigment Green (C.I. 74260)

5,4 g Wasser

100,0 g

**Beispiel 6: Das Haar blau anfärbendes Haarwachs**

42,2 g Polyethylenglykol (MG = 200)

37,0 g Polyethylenglykol (MG = 4000)

16,2 g hydriertes Rizinusöl, mit 40 bis 45
Mol Ethylenoxid oxethyliert (Cremophor® RH 40, BASF Aktiengesellschaft)

2,8 g C.I. Vat Blue 4 (C.I. 69 800)

1,8 g Wasser

100,0 g

**Beispiel 7: Dem Haar einen roten Perlglanz gebendes Haarwachs**

5

```
31,76 g Polyethylenglykol (MG = 200)

14,40 g Polyethylenglykol (MG = 4000)

13,84 g Glycerin

12,00 g hydriertes Rizinusöl, mit 40 bis 45
        Mol Ethylenoxid oxethyliert (Cremo-
        phor®  RH 40, BASF Aktiengesell-
        schaft)

20,00 g CTFA - Titandioxid/Mica/Carmin
        (Colorona®  Carmine Red No. 17272,
        E. Merck, Darmstadt)

 8,00 g Wasser

100,00 g
```

**Beispiel 8 Dem Haar einen goldenen Perlglanz gebendes Haarwachs**

```
31,76 g Polyethylenglykol (MG =200)

14,40 g Polyethylenglykol (MG = 4000)

13,84 g Glycerin

12,00 g hydriertes Rizinusöl, mit 40 bis 45
        Mol Ethylenoxid oxethyliert (Cremo-
        phor®  RH 40, BASF Aktiengesell-
        schaft)

20,00 g CTFA - Titandioxid/Mica/Eisenoxid
        (Timiron®  Gold Plus MP = 25
        No. 17 221, E. Merck, Darmstadt)

 8,00 g Wasser

100,00 g
```

Die Herstellung der Haarwachse gemäß Beispiel 1 bis 8 erfolgt, indem die Komponenten bei 65 Grad Celsius geschmolzen und miteinander vermischt werden. Anschließend wird die homogene Schmelze auf 40 Grad Celsius abgekühlt und in Tiegel abgefüllt.

**Beispiel 9: Vergleichsversuch**

Ein mit Wasser ausspülbares Haarwachs gemäß Beispiel 3 wurde im Halbseitenversuch mit einer wasserlöslichen Haarpomade nach Hugo Janistyn, "Handbuch der Kosmetika und Riechstoffe, Bd. III", Seite 325 (1973) der folgenden Zusammensetzung

```
60 g Polyethylenglykol (MG = 4000)

40 g Ethylhexandiol

100 g
```

EP 0 301 197 B1

an 14 dunkelhaarigen Versuchspersonen verglichen.

Das Haar wurde zunächst gewaschen und nach anschließender Trocknung in der Mitte gescheitelt. Die eine Hälfte des Haares wurde mit einem Haarwachs gemäß Beispiel 3 (Präparat A) und die andere Hälfte mit der gleichen Menge der vorstehend genannten Haarpomade nach Hugo Janistyn (Präparat B) behandelt.

Die Ergebnisse der anschließenden friseurtechnischen Beurteilung sind in den nachfolgenden Tabellen zusammengefaßt.

**Tabelle I: Auftrageeigenschaften**

| Bewertung | (Anzahl der Versuchspersonen) | | | |
|---|---|---|---|---|
| | sehr gut | gut | ausreichend | mangelhaft |
| Präparat A | 9 | 3 | 2 | 0 |
| Präparat B | 0 | 7 | 7 | 0 |

**Tabelle II: Frisurengestaltung/Haltbarkeit und Elastizität der Frisur**

| Bewertung | (Anzahl der Versuchspersonen) | | | |
|---|---|---|---|---|
| | sehr gut | gut | ausreichend | mangelhaft |
| Präparat A | 10 | 3 | 1 | 0 |
| Präparat B | 0 | 6 | 8 | 0 |

**Tabelle III: Glanz**

| Bewertung | (Anzahl der Versuchspersonen) | | | |
|---|---|---|---|---|
| | sehr gut | gut | ausreichend | mangelhaft |
| Präparat A | 12 | 2 | 0 | 0 |
| Präparat B | 0 | 4 | 1 | 9 |

Wie sich aus den Tabellen I bis III ersehen läßt, ist das erfindungsgemäße (mit Wasser leicht ausspülbare) Haarwachs der aus der Literatur bekannten wasserloslichen Haarpomade, sowohl bezüglich Glanz und Frisurengestaltung als auch bezuglich der Auftrageeigenschaften eindeutig überlegen.

**Patentansprüche**

1. Haarwachs, dadurch gekennzeichnet, daß es eine Kombination von
    (a) 10 bis 41 Gewichtsprozent Polyethylenglykol mit einem Molekulargewicht von 3000 bis 500,
    (b) 8 bis 25 Gewichtsprozent hydriertes Rizinusöl, welches mit 40 bis 60 Mol Ethylenoxid oxethyliert ist,
    (c) 36 bis 51 Gewichtsprozent Glycerin und/oder Ethylhexandiol und/oder Polyethylenglykol mit einem Molekulargewicht von 100 bis 300 und

7

(d) 0 bis 15 Gewichtsprozent Wasser.

enthält.

2. Haarwachs, dadurch gekennzeichnet, daß es eine Kombination von
   (a) 32 bis 41 Gewichtsprozent Polyethylenglykol mit einem Molekulargewicht von 3000 bis 5000,
   (b) 8 bis 25 Gewichtsprozent hydriertes Rizinusöl, welches mit 40 bis 45 Mol Ethylenoxid oxethyliert ist,
   (c) 36 bis 51 Gewichtsprozent Glycerin und/oder Ethylhexandiol und/oder Polyethylenglykol mit einem Molekulargewicht von 100 bis 300 und
   (d) 0 bis 10 Gewichtsprozent Wasser

   enthält.

3. Haarwachs, dadurch gekennzeichnet daß es eine Kombination von
   (a) 35 bis 39 Gewichtsprozent Polyethylenglykol mit einem Molekulargewicht von 4000,
   (b) 11 bis 18 Gewichtsprozent hydriertes Rizinusöl, welches mit 40 bis 45 Mol Ethylen oxid oxethyliert ist,
   (c) 39 bis 46 Gewichtsprozent Glycerin und/oder Ethylhexandiol und/oder Polyethylenglykol mit einem Molekulargewicht von 200 und
   (d) 2 bis 8 Gewichtsprozent Wasser

   enthält.

4. Haarwachs nach einem der Ansprüche 1 bis 3, dadurch gekennzeichnet, daß es 0,1 bis 6 Gewichtsprozent eines kosmetischen Haarfarbstoffes enthält.

5. Haarwachs nach Anspruch 4, dadurch gekennzeichnet, daß der kosmetische Haarfarbstoff ausgewählt ist aus C.I. Pigment Red 4 (C.I. 12 085), C.I. Pigment Green (C.I. 74 260) und C.I. Vat Blue 4 (C.I. 69 800).

6. Haarwachs nach einem der Ansprüche 1 bis 5, dadurch gekennzeichnet, daß es 1 bis 25 Gewichtsprozent eines Perlglanzpigmentes enthält.

7. Haarwachs, dadurch gekennzeichnet, daß es eine Kombination von
   (a) 10 bis 32 Gewichtsprozent Polyethylenglykol mit einem Molekulargewicht von 3000 bis 5000,
   (b) 8 bis 25 Gewichtsprozent hydriertes Rizinusöl, welches mit 40 bis 60 Mol Ethylenoxid oxethyliert ist
   (c) 36 bis 51 Gewichtsprozent Glycerin und/oder Ethylhexandiol und/oder Polyethylenglykol mit einem Molekulargewicht von 100 bis 300,
   (d) 0 bis 15 Gewichtsprozent Wasser und
   (e) 5 bis 25 Gewichtsprozent eines Perlglanzpigmentes

   enthält.

8. Haarwachs, dadurch gekennzeichnet, daß es eine Kombination von
   (a) 10 bis 25 Gewichtsprozent Polyethylenglykol mit einem Molekulargewicht von 4000,
   (b) 11 bis 18 Gewichtsprozent hydriertes Rizinusöl, welches mit 40 bis 45 Mol Ethylenoxid oxethyliert ist,
   (c) 39 bis 46 Gewichtsprozent Glycerin und/oder Ethylhexandiol und/oder Polyethylenglykol mit einem Molekulargewicht von 200,
   (d) 2 bis 12 Gewichtsprozent Wasser und
   (e) 10 bis 20 Gewichtsprozent eines Perlglanzpigmentes
   enthält.

9. Haarwachs nach einem der Ansprüche 6 bis 8, dadurch gekennzeichnet, daß das Perlglanzpigment ausgewählt ist aus CTFA-Titandioxid/Mica/Eisenoxid, CTFA-Titandioxid/Mica/Carmin, CTFA-Titandioxid/Mica/Eisenferrocyanid, CTFA-Titandioxid/Mica/Chromoxidgrün und CTFA-Titandioxid/Mica.

**Claims**

1. Hair wax, characterised in that it contains a combination of
   (a) 10 to 41 weight % of polyethylene glycol having a molecular weight of 3000 to 5000.
   (b) 8 to 25 weight % of hydrogenated castor oil, which is oxyethylated with 40 to 60 moles of ethylene oxide,
   (c) 36 to 51 weight % of glycerol and/or ethylhexanediol and/or polyethylene glycol having a molecular weight of 100 to 300 and
   (d) 0 to 15 weight % of water.

2. Hair wax, characterised in that it contains a combination of
   (a) 32 to 41 weight % of polyethylene glycol having a molecular weight of 3000 to 5000,
   (b) 8 to 25 weight % of hydrogenated castor oil, which is oxyethylated with 40 to 45 moles of ethylene oxide,
   (c) 36 to 51 weight % of glycerol and/or ethylhexanediol and/or polyethylene glycol having a molecular weight of 100 to 300 and
   (d) 0 to 10 weight % of water.

3. Hair wax, characterised in that it is a combination of
   (a) 35 to 39 weight % of polyethylene glycol having a molecular weight of 4000,
   (b) 11 to 18 weight % of hydrogenated castor oil, which is oxyethylated with 40 to 45 moles of ethylene oxide,
   (c) 39 to 46 weight % of glycerol and/or ethylhexanediol and/or polyethylene glycol having a molecular weight of 200 and
   (d) 2 to 8 weight % of water.

4. Hair wax according to any one of Claims 1 to 3, characterised in that it contains 0.1 to 6 weight % of a cosmetic hair dye.

5. Hair wax according to Claim 4, characterised in that the cosmetic hair dye is selected from C.I. Pigment Red 4 (C.I. 12 085), C.I. Pigment Green (C.I. 74 260) and C. I. Vat Blue 4 ( C.I. 69 800).

6. Hair wax according to any one of Claims 1 to 5, characterised in that it contains 1 to 25 weight % of a pearl gloss pigment.

7. Hair wax, characterised in that it contains a combination of
   (a) 10 to 32 weight % of polyethylene glycol having a molecular weight of 3000 to 5000,
   (b) 8 to 25 weight % of hydrogenated castor oil, which is oxyethylated with 40 to 60 moles of ethylene oxide,
   (c) 36 to 51 weight % of glycerol and/or ethylhexanediol and/or polyethylene glycol having a molecular weight of 100 to 300,
   (d) 0 to 15 weight % of water and
   (e) 5 to 25 weight % of a pearl gloss pigment.

8. Hair wax, characterised in that it contains a combination of
   (a) 10 to 25 weight % of polyethylene glycol having a molecular weight of 4000,
   (b) 11 to 18 weight % of hydrogenated castor oil, which is oxyethylated with 40 to 45 moles of ethylene oxide.
   (c) 39 to 46 weight % of glycerol and/or ethylhexanediol and/or polyethylene glycol having a molecular weight of 200.
   (d) 2 to 12 weight % of water and
   (e) 10 to 20 weight % of a pearl gloss pigment.

9. Hair wax according to any one of Claims 6 to 8, characterised in that the pearl gloss pigment is selected from CTFA-titanium dioxide/mica/iron oxide, CTFA-titanium dioxide/mica/carmine, CTFA-titanium dioxide /mica/iron ferrocyanide, CTFA-titanium dioxide/mica/chrome oxide green and CTFA-titanium dioxide/mica.

**Revendications**

1. Cire pour les cheveux caractérisée en qu'elle renferme une combinaison de :
   a) 10 à 41 % en poids de polyéthylène-glycol d'un poids moléculaire de 3.000 à 5.000,
   b) 8 à 25 % en poids d'huile de ricin hydrogénée, qui est éthoxylée avec 40 à 60 moles d'oxyde d'éhtylène,
   c) 36 à 51 % en poids de glycérol et/ou éthylhexane-diol, et/ou polyéthylène-glycol d'un poids moléculaire de 100 à 300, et
   d) 0 A 15 % en poids d'eau.

2. Cire pour les cheveux caractérisée en ce qu'elle renferme une combinaison de :
   a) 32 A 41 % en poids de polyéthylène-glycol d'un poids moléculaire de 3.000 à 5.000,
   b) 8 à 25 % en poids d'huile de ricin hydrogénée, qui est éthoxylée avec 40 à 45 moles d'oxyde d'éhtylène,
   c) 36 à 51 % en poids de glycérol et/ou ethylhexane-diol, et/ou polyéthylène-glycol d'un poids moléculaire de 100 à 300, et
   d) 0 à 10 % en poids d'eau.

3. Cire pour les cheveux, caractérisée en ce qu'elle renferme une combinaison de :
   a) 35 à 39 % en poids de Polyéthylène-glycol d'un poids moléculaire de 4.000,
   b) 11 à 18 % en poids d'huile de ricin hydrogénée, qui est éthoxylée avec 40 à 45 moles d'oxyde d'éhtylène,
   c) 39 à 46 % en poids de glycérol et/ou éthylhexane-diol, et/ou polyéthylène-glycol d'un poids moléculaire de 200, et
   d) 2 à 8 % en poids d'eau.

4. Cire pour les cheveux selon l'une des revendications 1 à 3, caractérisée en ce qu'elle renferme 0,1 à 6 % en poids d'un colorant cosmétique pour les cheveux.

5. Cire pour les cheveux selon la revendication 4, caractérisée en ce que le colorant cosmétique pour les cheveux est choisi parmi C.I. Pigment Red 4 (I.C. 12 085), C.I. Pigment Green (I.C. 74 260) et C.I. Vat Blue 4 (I.C. 69 800).

6. Cire pour les cheveux selon l'une des revendications 1 à 5, caractérisée en ce qu'elle renferme 1 à 25 % en poids d'un pigment donnant une coloration brillante perle.

7. Cire pour les cheveux, caractérisée en ce qu'elle renferme une combinaison de :
   a) 10 à 32 % en poids de polyéthylène-glycol d'un poids moléculaire de 3.000 à 5.000,
   b) 8 à 25 % en poids d'huile de ricin hydrogénée, qui est èthoxylée avec 40 à 60 moles d'oxyde d'éhtylène,
   c) 36 à 51 % en poids de glycérol et/ou éthylhexane-diol, et/ou polyéthylène-glycol d'un poids moléculaire de 100 à 300,
   d) 0 à 15 % en poids d'eau, et
   e) 5 à 25 % en poids d'un pigment donnant une coloration brillante perle.

8. Cire pour les cheveux, caractérisée en ce qu'elle renferme une combinaison de :
   a) 10 à 25 % en poids de polyéthylène-glycol d'un poids moléculaire de 4.000,
   b) 11 à 18 % en poids d'huile de ricin hydrogénée, qui est éthoxylée avec 40 à 45 moles d'oxyde d'éhtylène,
   c) 39 à 46 % en poids de glycérol et/ou éthylhexane-diol, et/ou polyéthylène-glycol d'un poids moléculaire de 200,
   d) 2 à 12 % en poids d'eau, et
   e) 10 à 20 % en poids d'un pigment donnant une coloration brillante perle.

9. Cire pour les cheveux selon l'une des revendications 6 à 8, caractérisée en ce que le pigment donnant une coloration brillante perle est choisi parmi les suivants : CTFA-dioxyde de titane/mica/oxyde de fer, CTFA-dioxyde de titane/mica/carmin, CTFA-dioxyde de titane/mica/ferrocyanure de fer, CTFA-dioxyde de titane/mica/vert d'oxyde de chrome et CTFA-dioxyde de titane/mica.